# EUROPEAN PATENT SPECIFICATION

(11) **EP 3 738 565 B1**
(45) Date of publication and mention of the grant of the patent: **09.08.2023**
(21) Application number: 19761140.3
(22) Date of filing: 19.02.2019
(51) Int. Cl.: A61F 13/56, A61F 13/49, A61F 13/494, A61F 13/53, A61F 13/513, A61F 13/514, A61F 13/537

(54) **LOW-WEIGHT INFANT DIAPER**
WINDEL FÜR KLEINKINDER MIT GERINGEM KÖRPERGEWICHT
COUCHE POUR NOURRISSONS DE FAIBLE POIDS

(30) Priority: 28.02.2018 JP 2018035603
(43) Date of publication of application: 18.11.2020
(73) Proprietor: Unicharm Corporation, Ehime 799-0111 (JP)
(72) Inventor: TSUJII, Maki, Kanonji-shi, Kagawa 769-1602 (JP); YAMANAKA, Yasuhiro, Kanonji-shi, Kagawa 769-1602 (JP); SAKAGUCHI, Satoru, Kanonji-shi, Kagawa 769-1602 (JP)
(74) Representative: Dolleymores
(86) International application number: PCT/JP2019/006118
(87) International publication number: WO 2019/167723

(56) References cited:
- WO-A1-2016/121236
- JP-A- H06 218 009
- JP-A- 2015 071 006

## Description

### [Technical Field]

The present invention relates to a low-weight infant diaper.

### [Background Art]

Open-type disposable diapers have conventionally been widely used. With an open-type disposable diaper, two end portions of belt members (e.g., fastening tape or straps) provided on the left and right sides of the diaper main body (e.g., an absorbent main body) are locked to the front side and the back side of the diaper main body in order to put on the diaper. Patent Document 1 discloses a disposable absorbent clothing article (diaper) that has straps (belt members) provided with fasteners (locking members) that can be detachably attached to both the back side and the front side.

### [Citation List]

### [Patent Literature]

[Patent Document 1] Japanese Patent Application Publication No. H6-218009 WO 2016/121236 A1 discloses a disposable diaper where a first waistline portion and a second waistline portion are connected by an engaging portion of a fastening tape engaging the first waistline portion. A cut-off line is formed for separating the fastening tape from the second waistline portion.

### [Summary of Invention]

### [Technical Problem]

According to the disposable diaper in Patent Document 1, the belt members can be attached and detached on the front side and the back side, thus facilitating the operations of putting on and taking off the diaper. For example, when a parent puts such a diaper on an infant, regardless of whether the infant is sleeping in a prone posture or a supine posture, the diaper can be easily put on or taken off on either the front side or the back side.

However, when the diaper is put on, if a portion of a locking member provided on a belt member peels away from the diaper main body, the detached portion of the locking member may become caught on and engaged with the infant's clothing or mat that they are sleeping on, for example, and the belt member may then completely separate from the diaper main body. Particularly in the case where the diaper is intended for a low-weight infant, movement of the low-weight infant's body needs to be minimized in order to reduce the burden placed thereon when the diaper is put on, and therefore if the belt member easily separates from the diaper main body, it becomes difficult to put on the diaper.

The present invention was achieved in light of conventional problems such as that described above, and an aspect of the present invention is to provide a diaper for a low-weight infant that suppresses the separation of a belt member from a main body portion when being put on.

A main aspect of the present invention for achieving the above-described aspect is a low-weight infant diaper having a longitudinal direction, a width direction, and a thickness direction that intersect each other,
the low-weight infant diaper including:
an absorbent body including at least an absorbent core;
an exterior sheet arranged on a non-skin side with respect to the absorbent body; and
a pair of belt members each of which includes a locking member on a one side in a longitudinal direction of the belt members and that includes a locking member on another side in the longitudinal direction of the belt members,
in a state where each of the belt members is locked, by the one-side locking member, to a non-skin side of the exterior sheet in the thickness direction at a location inward in the longitudinal direction with respect to an longitudinal outward end of the exterior sheet,
   an outward end of the absorbent core is located inward in the longitudinal direction with respect to a middle position,
   the middle position being a position between the longitudinal outward end of the exterior sheet and an outward end of the one-side locking member.

Features of the present invention other than the above will become clear by reading the description of the present specification with reference to the accompanying drawings.

### [Advantageous Effects of Invention]

According to the present invention, it is possible to provide a diaper for a low-weight infant that suppresses the separation of a belt member from a main body portion when being put on.

### [Brief Description of the Drawings]

FIG. 1 is a plan view of a diaper 1 in an unfolded and stretched state.
FIG. 2A is an exploded illustrative view of a schematic cross-section of a back portion 7 of the diaper 1. FIG. 2B is an exploded illustrative view of a schematic cross-section of a crotch portion 5 of the diaper 1.
FIGS. 3A and 3B are diagrams showing the diaper 1 in a put-on state.
FIGS. 4A and 4B are diagrams illustrating an operation of putting a diaper 100 according to a comparative example on a low-weight infant.
FIG. 5 is a diagram illustrating an operation of putting the diaper 1 on a low-weight infant.
FIG. 6 is a plan view illustrating the arrangement of a belt member 30 in the back portion 7 of the diaper 1.
FIG. 7 is a diagram illustrating a state in which an longitudinal outward end portion of a back sheet 24 has been folded.
FIG. 8 is a diagram illustrating a state in which the longitudinal outward end portion of the back sheet 24 has been folded in the case where the lock position of the belt member 30 of a conventional disposable diaper is unsuitable.
FIG. 9 is a diagram illustrating a folded state of the back sheet 24 in the case where an outward end 31Aleo of a locking member 31A is located outward in the longitudinal direction with respect to an outward end 21leo of an absorbent body 21.
FIG. 10 is a diagram illustrating a folded state of the back sheet 24 in the case where the absorbent body 21 has a core-wrapping sheet 21b.
FIG. 11 is a schematic cross-sectional view illustrating the thicknesses of portions of the diaper 1.
FIG. 12 is a plan view of a diaper 2 in an unfolded and stretched state.
FIG. 13 is an exploded illustrative view of a back portion 7 of the diaper 2.

### [Description of Embodiments]

At least the following matters will become clear with the description of this specification and the attached drawings.

A low-weight infant diaper having a longitudinal direction, a width direction, and a thickness direction that intersect each other,
the low-weight infant diaper including:
an absorbent body including at least an absorbent core;
an exterior sheet arranged on a non-skin side with respect to the absorbent body; and
a pair of belt members each of which includes a locking member on a one side in a longitudinal direction of the belt members and that includes a locking member on another side in the longitudinal direction of the belt members,
in a state where each of the belt members is locked, by the one-side locking member, to a non-skin side of the exterior sheet in the thickness direction at a location inward in the longitudinal direction with respect to an longitudinal outward end of the exterior sheet,
   an outward end of the absorbent core is located inward in the longitudinal direction with respect to a middle position,
   the middle position being a position between the longitudinal outward end of the exterior sheet and an outward end of the one-side locking member.

According to this low-weight infant diaper, when the diaper is put on a low-weight infant, the portion of the exterior sheet that is folded at a predetermined longitudinal position of the diaper covers the non-skin side of the outward ends of the one-side locking members (inward locking members). This makes it unlikely for the one-side locking members to become peeled away from the back sheet (main body portion). This therefore suppresses the separation of the belt members from the main body portion when the diaper is put on.

In such a low-weight infant diaper, it is desirable
that the absorbent body includes a core-wrapping sheet that covers the absorbent core, and
that an outward end of the core-wrapping sheet is located inward in the longitudinal direction with respect to the middle position,
   the middle position being the position between the longitudinal outward end of the exterior sheet and the outward end of the one-side locking member.

According to this low-weight infant diaper, when the diaper is put on a low-weight infant, even if the exterior sheet is folded at the outward end of the core-wrapping sheet, the longitudinal outward ends of the one-side locking members (inward locking members) are covered on the non-skin side. This makes it unlikely for the one-side locking members to become peeled away from the back sheet (main body portion). This therefore suppresses the separation of the belt member from the main body portions when the diaper is put on.

In such a low-weight infant diaper, it is desirable that
the longitudinal outward end of the absorbent core is located in the longitudinal direction between the outward end of the one-side locking member and the middle position,
the middle position being the position between the outward end of the exterior sheet and the outward end of the one-side locking member.

According to this low-weight infant diaper, when the diaper is put on a low-weight infant, the exterior sheet is likely to fold at the longitudinal outward end of the absorbent core (or core-wrapping sheet). The fold position of the exterior sheet is capable of being adjusted, thus making it more likely for the longitudinal outward end of the one-side locking member (inward locking member) to be covered. This therefore facilitates suppressing the separation of the belt members from the main body portion when the diaper is put on.

In such a low-weight infant diaper, it is desirable that
the outward end of the absorbent core is located inward in the longitudinal direction with respect to the outward end of the one-side locking member.

According to this low-weight infant diaper, the exterior sheet is likely to fold at the longitudinal outward end of the one-side locking member (inward locking member). The fold position of the exterior sheet is capable of being adjusted, thus making it more likely for the longitudinal outward end of the one-side locking member (inward locking member) to be covered. This therefore facilitates suppressing the separation of the belt members from the main body portion when the diaper is put on.

In such a low-weight infant diaper, it is desirable
that the low-weight infant diaper further comprises a leg elastic member capable of stretching and contracting in the longitudinal direction, and
that the one-side locking member is not overlapped in the longitudinal direction with a region in which the leg elastic member exhibits stretchability.

According to this low-weight infant diaper, in the region where the one-side locking member (inward locking member) is locked, the contractive force of the leg elastic member is less likely to act on the exterior sheet, and bending and wrinkling of the exterior sheet in such a region is suppressed. Accordingly, the one-side locking member is securely locked to the exterior sheet, and the separation of the belt members from the main body portion is further likely to be suppressed.

In such a low-weight infant diaper, it is desirable that
the one-side locking member is not overlapped in the width direction with an outward end of the absorbent body.

According to this low-weight infant diaper, the lock position of the one-side locking member (inward locking member) do not cross the portion where there is a large height difference at the widthwise outward end of the absorbent body, thus making it possible to suppress the case where the locking member easily become peeled away at such portion. Accordingly, the separation of the belt members from the main body portion is further likely to be suppressed.

In such a low-weight infant diaper, it is desirable that
an inward end of the one-side locking member is located outward in the width direction with respect to the outward end of the absorbent body.

According to this low-weight infant diaper, the one-side locking member (inward locking member) are not overlapped with the absorbent body. Accordingly, when, for putting the diaper on a low-weight infant, the diaper is inserted between the infant's body and a mat, suppressed is the case where the one-side locking member is pressed against the mat by the stiff absorbent body. This therefore facilitates suppressing the case where the one-side locking member becomes peeled away from the exterior sheet.

In such a low-weight infant diaper, it is desirable that
for, a total of a thickness of each of the belt members and a thickness of the one-side locking member is less than a thickness of the absorbent body.

According to this low-weight infant diaper, when the diaper is put on, a space is formed between a low-weight infant's body and a mat in accordance with the thickness of the absorbent body, and the belt member and the one-side locking member are likely to fit in the space. Accordingly, friction is less likely to occur between the belt member and the mat, and the case where the one-side locking member (inward locking member) become peeled away from the exterior sheet is likely to be suppressed.

In such a low-weight infant diaper, it is desirable
that the low-weight infant diaper further comprises a leg elastic member capable of stretching and contracting in the longitudinal direction,
that a widthwise distance between an outermost leg elastic member and a widthwise outward end of the exterior sheet is less than a width of the one-side locking member, and
that a widthwise inward end portion of the one-side locking member is located inward in the width direction with respect to the outermost leg elastic member.

According to this low-weight infant diaper, the one-side locking member (inward locking member) is less likely to protrude beyond the outermost outward end of the exterior sheet. Accordingly, the inward locking member is less likely to come into contact with a low-weight infant's skin, and it is possible to suppress the case of causing the low-weight infant to feel discomfort.

In such a low-weight infant diaper, it is desirable
that the low-weight infant diaper further comprises a pair of leak-proof walls on respective widthwise sides of the absorbent body,
that each of the leak-proof walls includes an LSG elastic member capable of stretching and contracting in the longitudinal direction, and
that in a state where the low-weight infant diaper has been unfolded and stretched,
   the one-side locking member is located outward in the width direction with respect to the LSG elastic members.

According to this low-weight infant diaper, suppressed is the case where the one-side locking member (inward locking member) is locked at a position that is too far inward in the width direction. Accordingly, when the diaper is put on a low-weight infant, it is possible to suppress the occurrence of problems such as that the longitudinal length of the belt members is insufficient, and that the other-side locking member (outward locking member) cannot be locked to the exterior sheet.

### Embodiments

### Basic configuration of disposable diaper 1

A disposable diaper 1 (hereinafter also simply called the diaper 1) of the present embodiment is a low-weight infant disposable diaper that is mainly intended for a newborn infant or an infant, and is favorably for use by a low-weight infant, whose body weight is 3000 g or less, and particularly favorably for use by a low-birth-weight infant, whose body weight is less than 2500 g. Note that the concept of a low-weight infant includes not only a low-birth-weight infant (having a body weight less than 2500 g), but also a very-low-birth-weight infant (having a body weight less than 1500 g) and an extremely-low-birth-weight infant (having a body weight less than 1000 g) .

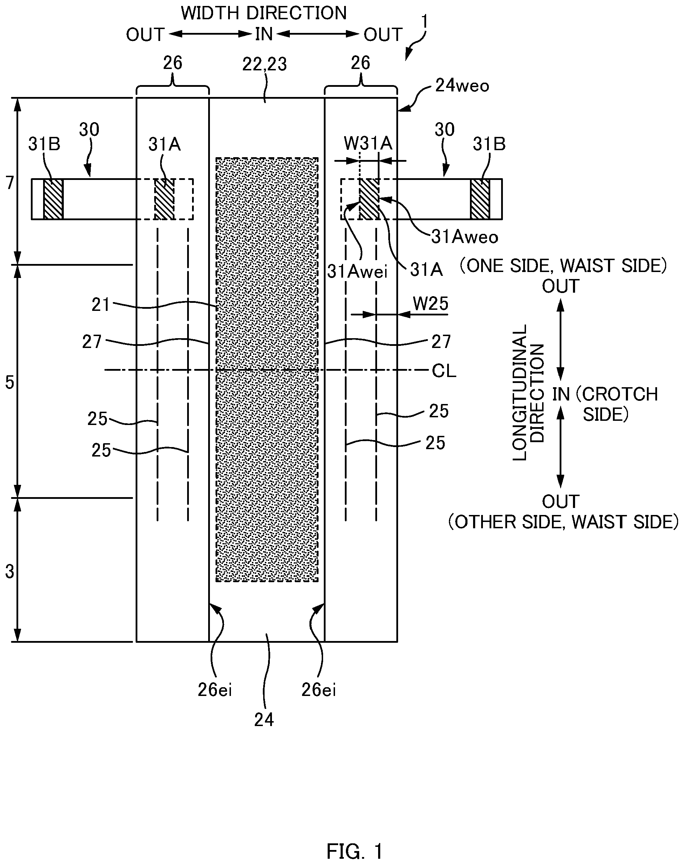

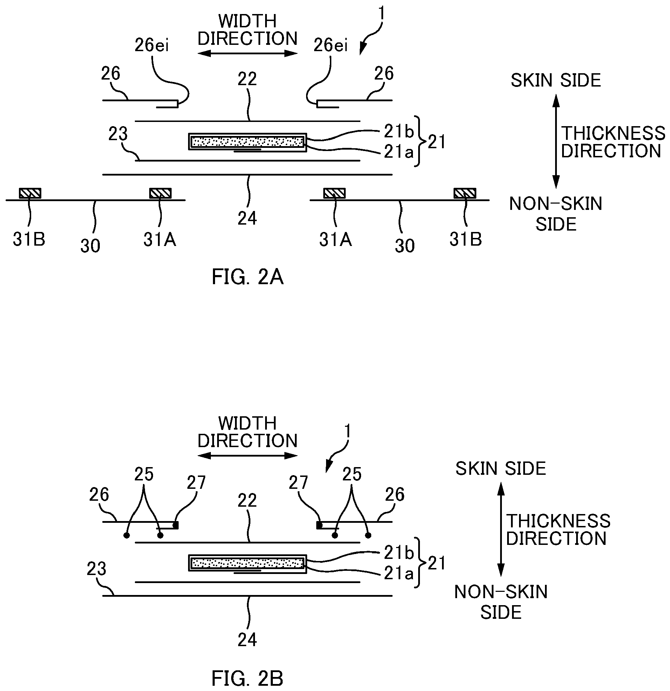

FIG. 1 is a plan view of the diaper 1 in an unfolded and stretched state. FIG. 2A is an exploded illustrative view of a schematic cross-section of a back portion 7 of the diaper 1. FIG. 2B is an exploded illustrative view of a schematic cross-section of a crotch portion 5 of the diaper 1. Note that the unfolded and stretched state of the diaper 1 refers to a state in which the diaper 1 has been unfolded and stretched such that wrinkles in the diaper 1 are substantially no longer visible, that is to say a state in which the diaper 1 has been stretched such that the lengths of constituent members of the diaper 1 (e.g., a later-described top sheet 22 and the like) match or are close to the dimensions of the members on their own.

The diaper 1 of the present embodiment is a so-called open-type disposable diaper, and as shown in FIG. 1, includes a front portion 3, a crotch portion 5, and a back portion 7. The front portion 3 is the portion that is located on the front portion side (front side, front waist side) of the wearer. Also, the back portion 7 is the portion that is located on the back portion side (back side, back waist side) of the wearer. Note that the front portion 3 and the back portion 7 are defined in this way for the sake of convenience, but the front portion 3 and the back portion 7 can be switched when the diaper 1 is used (worn). In other words, the back portion 7 may be located on the front side of the wearer, and the front portion 3 may be located on the back side of the wearer. The crotch portion 5 is the portion that is located between the front portion 3 and the back portion 7.

As shown in FIG. 1, the directions used in the following descriptions are defined as follows. The direction from the front portion 3 toward the back portion 7 is the "longitudinal direction", and the "width direction" is a direction orthogonal to the longitudinal direction. The line CL in FIG. 1 denotes a line that indicates the center of the diaper 1 in the longitudinal direction. Also, as shown in FIG. 2, the "thickness direction" is a direction orthogonal to the longitudinal direction and the width direction, and in this direction, the side corresponding to the wearer's skin is the "skin side", and the side opposite thereto is the "non-skin side".

The diaper 1 includes a main body portion 10 and belt members 30. Note that although FIG. 1 shows a state in which the belt members 30 are locked to the main body portion 10, the belt members 30 can be attached to and detached from the main body portion 10.

### Main body portion 10

The main body portion 10 is the portion that absorbs and holds a fluid such as urine excreted by the wearer, and as shown in FIG. 1, is constituted by the front portion 3, the crotch portion 5, and the back portion 7, and is substantially rectangular in a plan view. The main body portion 10 mainly includes an absorbent body 21, a top sheet 22, a leak-proof sheet 23, a back sheet 24, and side sheets 26 (see FIGS. 2A and 2B).

The absorbent body 21 includes an absorbent core 21a constituted by layers of a liquid absorbent material that can absorb excrement such as urine, and is arranged extended over the front portion 3, the crotch portion 5, and the back portion 7. The dotted region in FIG. 1 indicates the region occupied by the absorbent core 21a. Hereinafter, in this specification, the absorbent body 21 and the absorbent core 21a are considered to mean the same thing, unless particularly stated otherwise. Note that the absorbent body 21 may be configured such that the absorbent core 21a is covered by a core-wrapping sheet 21b. The absorbent body 21 (absorbent core 21a) of the present embodiment is substantially rectangular and elongated in the longitudinal direction. Note that the shape of the absorbent body 21 is not intended to be limited to the shape shown in FIG. 1, and need only be provided in at least the crotch portion 5.

The absorbent body 21 of the present embodiment is arranged so as to be sandwiched between the top sheet 22 and the leak-proof sheet 23 in the thickness direction. The liquid absorbent material that constitutes the absorbent core 21a can be made of liquid absorbent fibers such as pulp fibers, or liquid-absorbent particulate matter such as a superabsorbent polymer. The liquid absorbent material may include a liquid absorbent material other than liquid absorbent fibers and liquid-absorbent particulate matter. The member constituting the core-wrapping sheet 21b can be made of tissue paper, nonwoven fabric, or the like.

The top sheet 22 is a liquid-permeable member arranged on the skin side of the absorbent body 21, and is made of nonwoven fabric, for example. The leak-proof sheet 23 is a liquid-impermeable member arranged on the non-skin side of the absorbent body 21, and is constituted by a resin film sheet, for example. The back sheet 24 is a member (exterior sheet) that constitutes the exterior of the non-skin side of the diaper 1, and is made of nonwoven fabric, for example. The back sheet 24 is arranged on the non-skin side of the leak-proof sheet 23 (see FIGS. 2A and 2B).

At least the crotch portion 5 of the main body portion 10 is provided with leg elastic members 25 (e.g., elastic strings; see FIG. 2B) that can stretch and contract in the longitudinal direction and are arranged between the side sheets 26 and the back sheet 24 (the top sheet 22 in FIG. 2B). The leg elastic members 25 are members that give the main body portion 10 stretchability in the crotch portion 5. In the present embodiment, the leg elastic members 25 are attached in a state of being stretched in the longitudinal direction. The leg elastic members 25 thus give longitudinal stretchability within a range indicated by dashed lines in FIG. 1. When the diaper 1 is put on, the crotch portion 5 contracts along the longitudinal direction, and thus the main body portion 10 three-dimensionally deforms into a rounded shape that curves along the wearer's body, the two widthwise side portions of the main body portion 10 contract, and leg gathers are formed around the wearer's legs. Accordingly, the main body portion 10 is held in a shape that wraps around the wearer's crotch, thus improving the fit of the diaper 1. This also facilitates suppressing the case where excrement leaks from around the wearer's legs to the outside of the diaper 1.

The side sheets 26 are a pair of members provided on the skin side in the thickness direction with respect to the top sheet 22 and on the two widthwise sides of the absorbent body 21, and are made of nonwoven fabric, for example. In widthwise outward end regions, the side sheets 26 are joined to the top sheet 22 and the back sheet 24 in the thickness direction. Also, as shown in FIGS. 2A and 2B, the side sheets 26 are folded outward at widthwise inward ends 26ei such that the folded portions are overlaid thereon in the thickness direction. Between the portions of the folded side sheets 26 in the thickness direction, provided are LSG elastic members 27 (e.g., elastic strings) that can stretch and contract in the longitudinal direction. The LSG elastic members 27 are members that give the side sheets 26 stretchability in the crotch portion 5. In the present embodiment, the LSG elastic members 27 are attached in a state of being stretched in the longitudinal direction, and the LSG elastic members 27 exhibit contractive force in the longitudinal direction. When the diaper 1 is put on, the LSG elastic members 27 contract in the longitudinal direction, and thus the portions of the side sheets 26 that are not joined to the top sheet 22 (back sheet 24) rise up toward the wearer's skin, and the side sheets 26 function as a pair of leak-proof walls that suppress the lateral leakage of excrement or the like.

### Belt members 30

Two belt members 30 are respectively arranged at the two widthwise side portions of the main body portion 10 in the back portion 7 (back side) of the diaper 1. As shown in FIG. 1, the belt members 30 of the present embodiment are band members that are substantially rectangular and elongated in the width direction of the diaper 1. The belt members 30 each include a locking member 31 on each longitudinal side thereof (hereinafter, also called the belt longitudinal direction), and the locking members 31 can be locked to the exterior sheet (back sheet 24) of the diaper 1. The locking members 31 are members having hooks that project in the thickness direction, and are constituted by hook-and-loop fasteners or the like. The hooks (not shown) of the locking members 31 become caught on the fibers at the surface of the nonwoven fabric that constitutes the exterior sheet (back sheet 24) of the diaper 1, and thus the belt members 30 are locked to the main body portion 10 (exterior sheet) via the locking member 31.

As described above, the belt member 30 of the present embodiment includes one locking member 31 in the end portion on one side in the belt longitudinal direction (the inward side in the width direction of the diaper 1) and one locking member 31 in the end portion on the other side (the outward side in the width direction of the diaper 1). Accordingly, both sides of the belt members 30 in the belt longitudinal direction can be locked to exterior sheet (back sheet 24) of the diaper 1. Hereinafter, the locking member 31 provided on the one side in the belt longitudinal direction (in FIG. 1, the inward side in the width direction of the diaper 1) will also be called an inward locking member 31A, and the locking member 31 provided on the other side in the belt longitudinal direction (in FIG. 1, the outward side in the width direction of the diaper 1) will also be called an outward locking member 31B. The diaper 1 is described below in the state where the belt members 30 have been locked to the non-skin side of the back sheet 24 (exterior sheet) with use of the inward locking members 31A as shown in FIGS. 1 and 2.

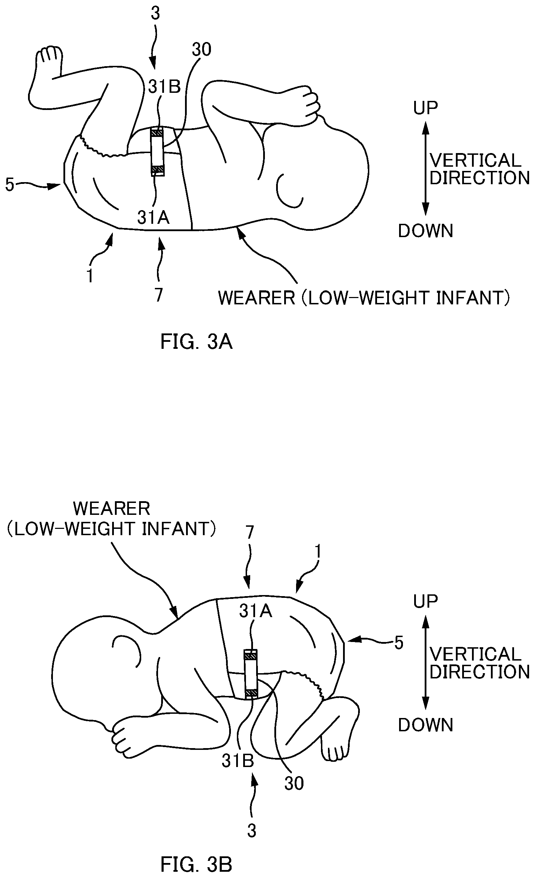

When the diaper 1 is put on, the outward locking members 31B of the belt members 30 are locked to the non-skin side of the back sheet 24 (exterior sheet) in the front portion 3 (front side) of the main body portion 10, thus forming a waist opening and a pair of leg openings of the diaper 1, and therefore the position of the diaper 1 can be fixed on the wearer's body (waist) (see FIGS. 3A and 3B). Note that the front portion 3 of the diaper 1 may be provided with target tape (not shown), which is for facilitating the locking of the outward locking members 31B, in a region that is on the non-skin side in the thickness direction in the widthwise central portion of the back sheet 24. Also, a configuration is possible in which, when the diaper 1 is put on, the waist opening is formed by the outward locking member 31B of the widthwise one-side belt member 30 being locked to the non-skin-side surface of the widthwise other-side belt member 30.

The longitudinal product length of the diaper 1 for a low-weight infant of the present embodiment (the dimension in the state where the product is stretched in the longitudinal direction so as to eliminate wrinkles) is in the range of 170 to 330 mm. For example, the product length of the diaper 1 is 310 mm in the case of being for a low-birth-weight infant having a body weight less than 2500 g, the product length of the diaper 1 is 270 mm in the case of being for a very-low-birth-weight infant having a body weight less than 1500 g, and the product length of the diaper 1 is 170 mm in the case of being for an extremely-low-birth-weight infant having a body weight less than 1000 g.

Also, the waist dimension of the diaper 1 for a low-weight infant of the present embodiment is in the range of 160 to 295 mm. Note that the waist dimension is the dimension in the state where the widthwise outward end portion of one of the belt members 30 is aligned with the widthwise inward end portion of the other belt member 30, and the product is stretched to eliminate wrinkles. In other words, it is a dimension in the state where the product is stretched in the width direction. For example, the waist dimension of the diaper 1 is 273.5 mm in the case of being for a low-birth-weight infant, and the waist dimension of the diaper 1 is 220 mm in the case of being for a very-low-birth-weight infant having a body weight less than 1500 g.

### Operation of putting on diaper 1

The following describes operations when putting the diaper 1 on a low-weight infant who is the intended wearer. FIGS. 3A and 3B are diagrams showing the diaper 1 in a put-on state.

When an open-type disposable diaper such as the diaper 1 is put on, first, the back portion 7 of the diaper 1 in the unfolded state is arranged at the back part of the intended wearer, the crotch portion 5 is placed against the wearer's crotch part, and the front portion 3 is placed against the wearer's stomach part. Then the pair of belt members 30, which are locked to the two widthwise sides of the main body portion 10 in the back portion 7 (back side), are wrapped around the wearer's waist from the back side toward the front side, and the outward locking members 31B are locked to the non-skin side of the front portion 3 (front side) so as to fix the diaper 1 around the wearer's waist. The diaper 1 is can thus be put on the intended wearer as shown FIG. 3A. Conversely, when the diaper 1 is taken off, the operations are performed in the reverse of when being put on, that is to say, the outward locking members 31B, which are locked to the back sheet 24 (exterior sheet) in the front portion 3 (front side), are peeled off to release the waist portion, and the diaper 1 can thus being taken off easily.

Note that low-weight infants, which are the intended wearer of the diaper 1 of the present embodiment, are generally placed in an incubator to grow in a hospital or the like. For this reason, when the diaper for a low-weight infant is put on or taken off, the worker (person who is putting the diaper on the low-weight infant or taking it off) needs to put their hand through an operation window provided in the incubator and perform the task in a small space. Also, it is common for the low-weight infant to be placed in the incubator in a prone posture in which their back is curved in a C shape and their legs are sharply bent into an M shape, as shown in FIG. 3B. This posture will also be called the "positioning posture", which is a posture that is close to the posture taken in the mother's womb (fetus-like bent posture) and is a calming posture. When the low-weight infant is in the positioning posture, the outward locking members 31B are located below the stomach of the wearer (low-weight infant) as shown in FIG. 3B.

Accordingly, conventionally, when performing the task of changing the diaper on a low-weight infant that is in the positioning posture in an incubator, the worker has needed to put their hand under the low-weight infant's stomach inside the small space and peel off the outward locking members 31B of the belt members 30. In contrast, with the diaper 1 of the present embodiment, the inward locking members 31A of the belt members 30 can be peeled off the back sheet 24 (exterior sheet). For this reason, as shown in FIG. 3B, the worker can easily peel off the inward locking members 31A on the back side of the low-weight infant. Accordingly, the diaper can be changed without subjecting the low-weight infant to the burden of a change in posture, for example. Furthermore, the diaper 1 of the present embodiment can be put on backwards such that the front portion 3 is on the back side and the back portion 7 is on the front side, and therefore regardless of the posture of the low-weight infant when sleeping, the diaper 1 can be easily put on or taken off without moving the low-weight infant's body, thus making the diaper 1 favorable for low-weight infants.

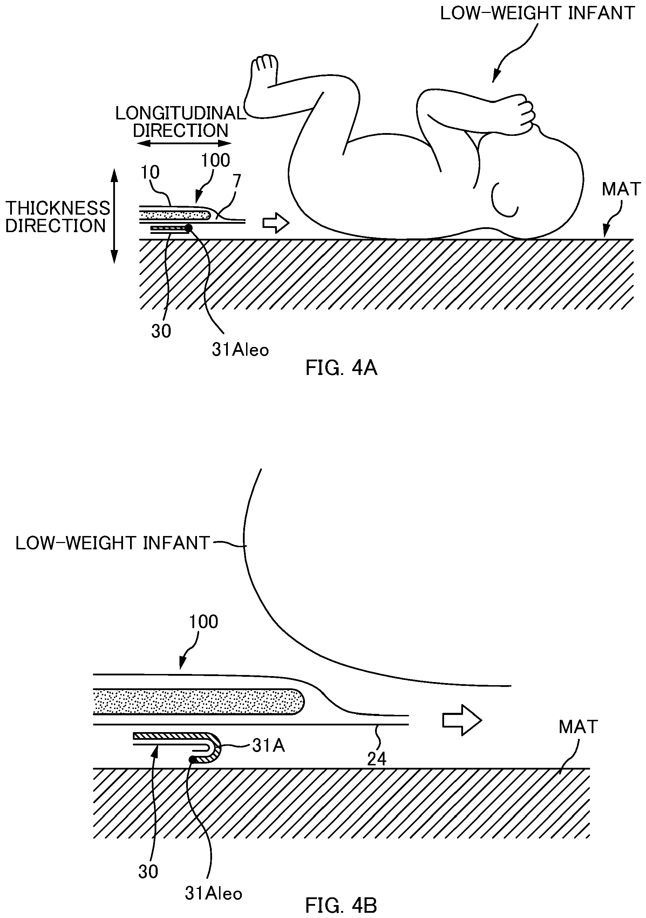

Also, in the case of a diaper having a structure in which the belt members are locked to the main body portion with use of locking members on the two widthwise sides (belt longitudinal direction), as with the diaper 1, sometimes the belt members become detached from the main body portion due to the locking members locked to the main body portion becoming unintendedly peeled off when the diaper is put on. FIGS. 4A and 4B are diagrams illustrating an operation of putting a diaper 100 according to a comparative example on a low-weight infant. The configuration of the diaper 100 according to the comparative example is approximately the same as that of the diaper 1 of the present embodiment, with the exception of a difference in the lock positions of the belt members 30. Also, in the diaper 100, the belt members 30 are simply locked to the exterior sheet (back sheet 24) with use of the inward locking members 31A, which are hook-and-loop fasteners or the like, and the belt members 30 (inward locking members 31A) can be easily peeled away from the exterior sheet.

FIG. 4A is a side view of the case where the diaper 100 is put on a low-weight infant who is sleeping face-up on a mat. First, the back portion 7 of the diaper 100 is arranged at the back part of the low-weight infant. Specifically, the back portion 7 of the unfolded diaper 100 is arranged between the low-weight infant's body and the mat by being inserted under the low-weight infant's crotch. At this time, in order to reduce the burden placed on the low-weight infant's body, rather than lifting up the low-weight infant's body, the diaper 100 is pressed downward toward the mat (i.e., vertically downward) while the back portion 7 of the diaper 100 is inserted between the low-weight infant's back part and the mat.

Due to the diaper 100 being pressed against the mat, frictional force is likely to act between the belt members 30 and the mat, and outward ends 31Aleo of the inward locking members 31A become curled up in the longitudinal direction as shown in FIG. 4B, and the curled portions become peeled away from the back sheet 24 (exterior sheet) . The curled portions of the inward locking members 31A that have been peeled away from the exterior sheet (the portions including the outward ends 31Aleo) may then engage with the mat as shown in FIG. 4B. If the diaper 100 is further inserted under the low-weight infant's back portion in this state, there is a risk as follow: the inward locking members 31A become entirely peeled away from the back sheet 24, and the belt members 30 become entirely separated from the main body portion 10. If the belt members 30 become separated, the locking members 31 need to be re-locked to the main body portion 10 (back sheet 24), and the operation shown in FIG. 4A needs to be performed again, thus placing a burden on the low-weight infant's body.

### Arrangement of belt members 30

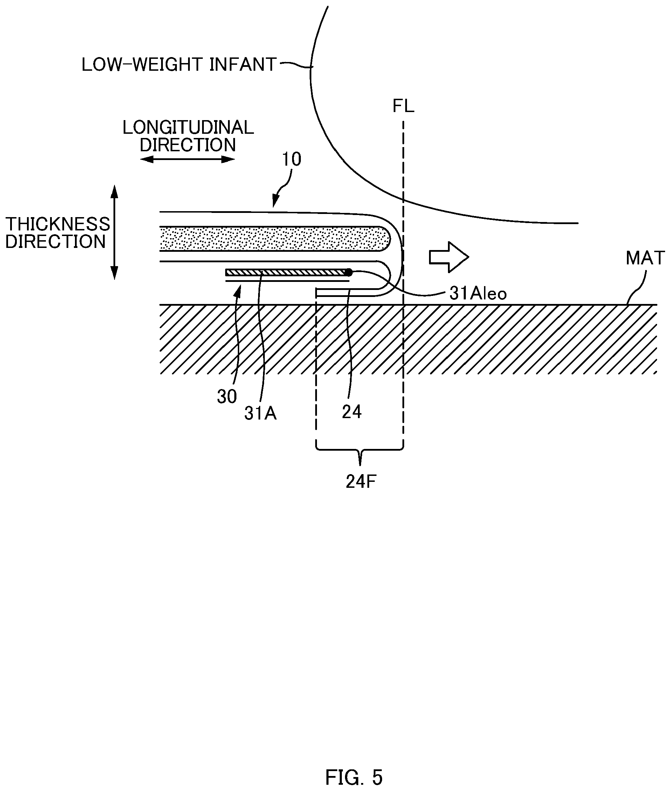

In contrast, with the diaper 1 of the present embodiment, by appropriately adjusting the positions at which the belt members 30 are locked, it is possible to suppress the separation of the belt members 30 from the main body portion 10 when the diaper 1 is put on. FIG. 5 is a diagram illustrating the operation of putting the diaper 1 of the present embodiment on a low-weight infant, and corresponds to FIG. 4B that illustrates the operation of putting on the diaper 100 of the comparative example.

In the case of putting the diaper 1 on a low-weight infant who is sleeping in a supine posture, similarly to the case described using FIG. 4, the back portion 7 of the diaper 1 in the unfolded state is arranged between the low-weight infant's body and the mat by being inserted under the low-weight infant's crotch. At this time, in the diaper 1, an outward end portion of the back sheet 24 (exterior sheet) in the longitudinal direction folds to the non-skin side in the thickness direction at a predetermined fold position FL. A folded portion 24F becomes folded over the non-skin side of the outward end portion of the inward locking members 31A. Specifically, as shown in FIG. 5, the non-skin side of the longitudinal outward end 31Aleo of the inward locking member 31A is covered by the folded portion 24F of the back sheet 24 (exterior sheet). Accordingly, frictional force is not generated between the outward end 31Aleo of the inward locking member 31A and the mat, suppressing the peeling of the outward end 31Aleo away from the back sheet 24 (exterior sheet). The belt member 30 is therefore not likely to become separated from the main body portion 10 (back sheet 24).

Note that in FIG. 5, the top sheet 22 is folded at the fold position FL together with the back sheet 24, and is folded over the non-skin side of the outward end 31Aleo, but it is sufficient that any one or more of the constituent members of the main body portion 10 (e.g., the top sheet 22, the leak-proof sheet 23, and the back sheet 24) is folded so as to cover the non-skin side of the outward end 31Aleo. In the following description, It is assumed that at least the longitudinal outward end portion of the back sheet 24 (exterior sheet) is folded at the fold position FL.

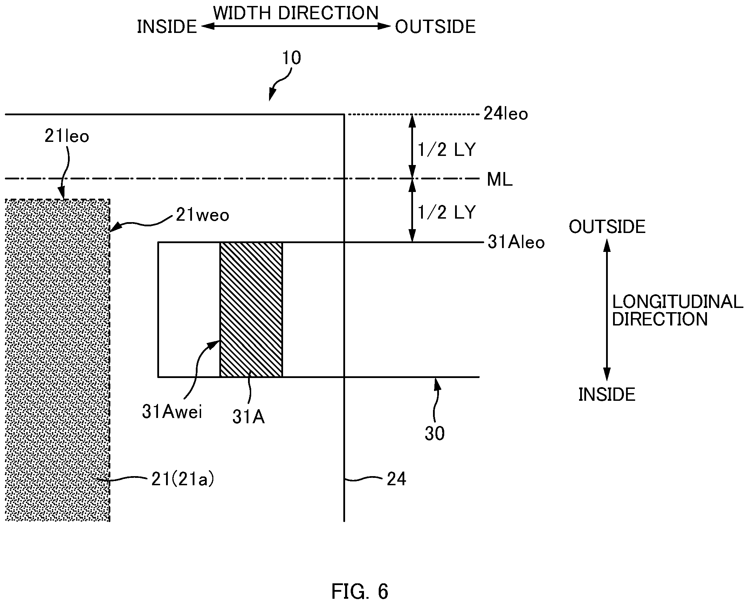

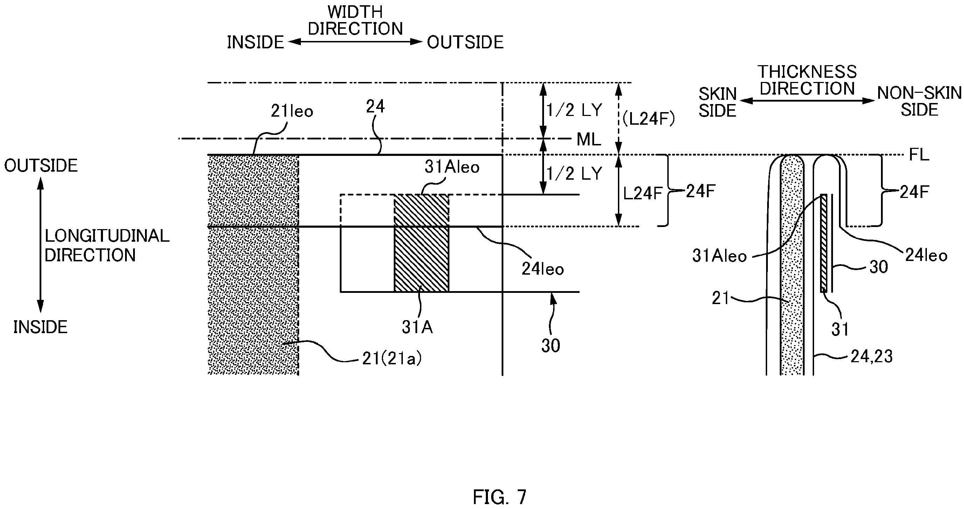

FIG. 6 is a plan view illustrating the arrangement of the belt member 30 in the back portion 7 of the diaper 1. FIG. 6 shows the positional relationship with the absorbent body 21 and the back sheet 24 (exterior sheet), centered on the inward locking member 31A of the belt member 30 arranged on the right side in the width direction in FIG. 1. FIG. 7 is a diagram illustrating a state in which the longitudinal outward end portion of the back sheet 24 has been folded.

As shown in FIG. 6, a middle position ML is a position at the middle between the longitudinal outward end 24leo of the back sheet 24 (exterior sheet) and the longitudinal outward end 31Aleo of the inward locking member 31A. Also, a distance LY is the distance between the longitudinal outward end 24leo and the longitudinal outward end 31Aleo. In the diaper 1 of the present embodiment, the belt member 30 is arranged (locked) such that the outward end 21leo of the absorbent body 21 is inward in the longitudinal direction with respect to the middle position ML.

When the diaper 1 in the state shown in FIG. 6 is put on a low-weight infant, the longitudinal outward end portion of the back sheet 24 (exterior sheet) folds to the non-skin side at the predetermined fold position FL, as described using FIG. 5. The folded portion 24F thus covers the non-skin side of the outward end 31Aleo of the inward locking member 31A, and the inward locking member 31A is not likely to become peeled away from the back sheet 24.

Here, in order for the folded portion 24F of the back sheet 24 to be overlaid in the longitudinal direction on the non-skin side of the outward end 31Aleo of the inward locking member 31A, the fold position FL needs to be inward in the longitudinal direction with respect to the middle position ML. In other words, the back sheet 24 needs to be folded at a position inward in the longitudinal direction with respect to the middle position ML.

If the back sheet 24 is folded at the middle position ML (i.e., the middle position ML and the fold position FL are the same position), the outward end 24leo of the folded portion of the back sheet 24 becomes located at the same longitudinal position as the outward end 31Aleo of the locking member 31A. In other words, a longitudinal length L24F of the folded portion 24F of the back sheet 24 is equivalent to 1/2 the longitudinal distance LY between the outward end 24leo of the back sheet 24 and the outward end 31Aleo of the locking member 31A. In contrast, if the fold position FL is inward in the longitudinal direction with respect to the middle position ML, the longitudinal length L24F of the folded portion 24F of the back sheet 24 is larger than 1/2 the distance LY, and the folded portion 24F is reliably overlaid on the outward end 31Aleo.

In FIG. 7, the "fold position FL" for folding of the back sheet 24 is located at the position of the longitudinal outward end 21leo of the absorbent body 21 (absorbent core 21a). Because the absorbent core 21a is constituted by layers of a liquid absorbent material such as pulp fibers that are overlaid in the thickness direction, the absorbent core 21a has a higher stiffness than the back sheet 24 and the top sheet 22 (i.e., nonwoven fabric sheets). For this reason, in the longitudinal direction, the main body portion 10 has a large change in stiffness at the boundary with the outward end 21leo of the absorbent body 21 (absorbent core 21a), and the back sheet 24 becomes more likely to be folded at the outward end 21leo at which the difference in stiffness is large.

Accordingly, in the diaper 1 of the present embodiment, the fold position (i.e., the position of the outward end 21leo) is located inward in the longitudinal direction with respect to the middle position ML. The folded portion 24F of the back sheet 24 therefore covers the non-skin side of the outward end 31Aleo of the inward locking member 31A, and, when the diaper 1 is put on, the inward locking member 31A is not likely to become peeled away, and the separation of the belt member 30 from the main body portion 10 is suppressed.

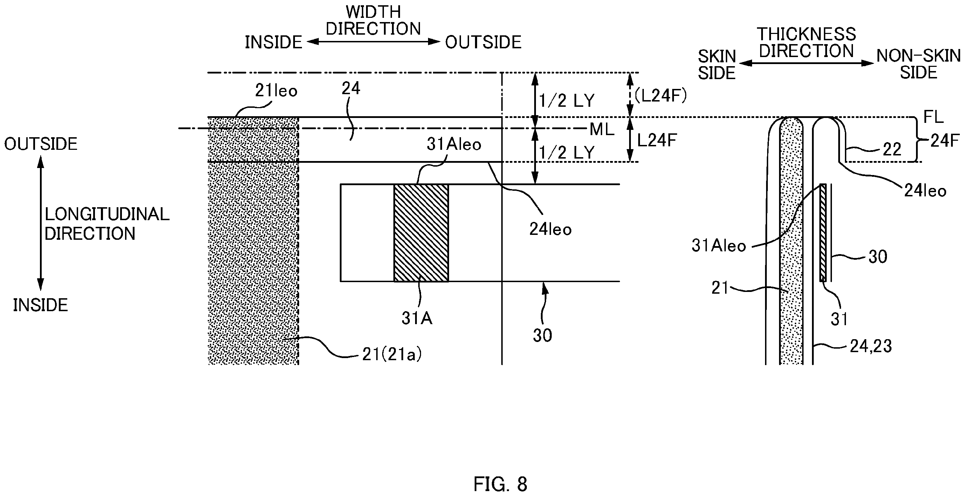

FIG. 8 is a diagram illustrating a state in which the longitudinal outward end portion of the back sheet 24 has been folded in the case where the lock position of the belt member 30 of a conventional disposable diaper (e.g., including the diaper 100 of the comparative example described above) is unsuitable. In FIG. 8, the belt member 30 (inward locking member 31A) is arranged such that the outward end 21leo of the absorbent body 21 is outward in the longitudinal direction with respect to the middle position ML. In this case, the longitudinal length L24F of the folded portion 24F of the back sheet 24 is smaller than 1/2 the distance LY, and the folded portion 24F is not overlaid on the outward end 31Aleo in the longitudinal direction. Accordingly, when the diaper is put on the intended wearer, similarly to the case described using FIG. 4B, there is a risk that the outward end 31Aleo easily becomes peeled away from the back sheet 24 (exterior sheet) and the belt member 30 become separated from the main body portion 10. In contrast, according to the diaper 1 of the present embodiment, when the back sheet 24 is folded, the situation shown in FIG. 8 does not arise, thus making it possible to effectively suppress the separation of the belt member 30.

Note that FIG. 7 shows the case where the outward end 21leo of the absorbent body 21 is located in a longitudinal region that is between the middle position ML and the outward end 31Aleo of the locking member 31A. Specifically, because the outward end 21leo of the absorbent body 21 is located inward in the longitudinal direction with respect to the middle position ML and outward in the longitudinal direction with respect to the outward end 31Aleo of the locking member 31A, the back sheet 24 has been folded at the outward end 21leo (fold position FL) of the absorbent body 21 (absorbent core 21a). However, the outward end 21leo of the absorbent body 21 may be located inward in the longitudinal direction with respect to the middle position ML and inward in the longitudinal direction with respect to the outward end 31Aleo.

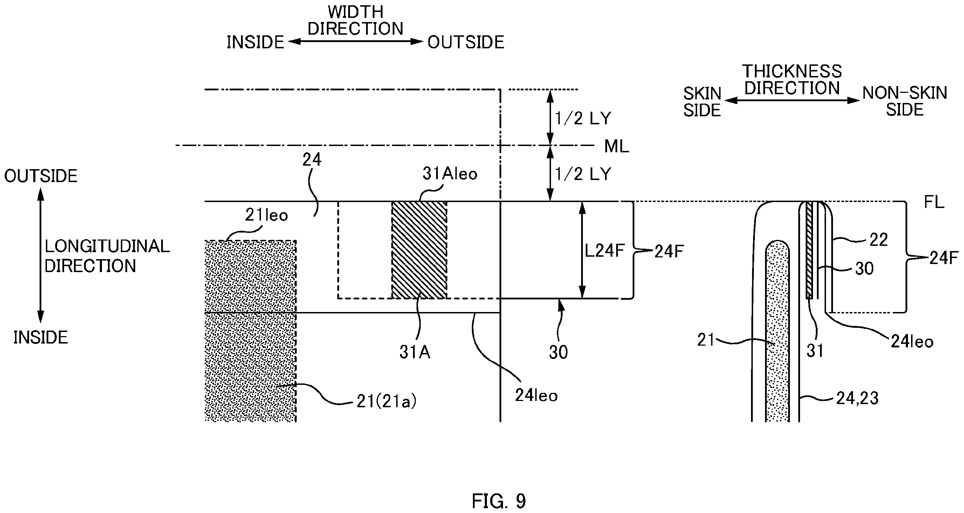

FIG. 9 is a diagram illustrating a folded state of the back sheet 24 in the case where the outward end 31Aleo of the locking member 31A is located outward in the longitudinal direction with respect to the outward end 21leo of the absorbent body 21. In the case shown in FIG. 9, the fold position FL of the back sheet 24 is located at the position of the longitudinal outward end 31Aleo of the inward locking member 31A, not the position of the longitudinal outward end 21leo of the absorbent body 21. The locking member 31 has a high stiffness due to being a hook-and-loop fastener that includes hooks or the like. Therefore, in the longitudinal direction, the difference in stiffness is large at the position of the outward end 31Aleo. For this reason, the fold position FL is formed at the position of the longitudinal outward end 31Aleo of the locking member 31A, and the back sheet 24 (exterior sheet) becomes folded at the fold position FL. In this case as well, the non-skin side of the outward end 31Aleo of the inward locking member 31A is covered by the folded portion 24F of the back sheet 24, and, when the diaper 1 is put on, the inward locking member 31A is not likely to become peeled away, and the separation of the belt member 30 from the main body portion 10 is suppressed.

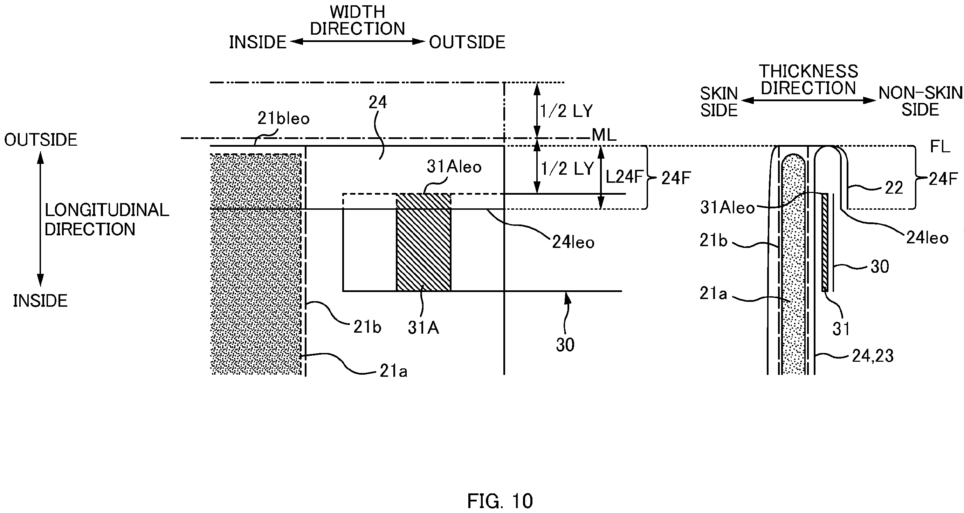

Also, there are cases where the absorbent body 21 of the diaper 1 includes the core-wrapping sheet 21b that wraps around the absorbent core 21a, as described above. An outward end 21bleo of the core-wrapping sheet 21b may be located outward of the outward end 21leo of the absorbent core 21a in the longitudinal direction. FIG. 10 is a diagram illustrating a folded state of the back sheet 24 in the case where the absorbent body 21 includes the core-wrapping sheet 21b.

In the case shown in FIG. 10, in the longitudinal direction, the difference in stiffness is large at the position of the outward end 21bleo of the core-wrapping sheet 21b, and therefore the fold position FL of the back sheet 24 is formed at the outward end 21bleo. Accordingly, if the outward end 21bleo of the core-wrapping sheet 21b is inward in the longitudinal direction with respect to the middle position ML, similarly to the case described above, the non-skin side of the outward end 31Aleo of the inward locking member 31A is covered by the folded portion 24F of the back sheet 24. Accordingly, when the diaper 1 is put on, the inward locking member 31A is not likely to become peeled away, and the separation of the belt member 30 from the main body portion 10 is suppressed. Note that if the difference in stiffness is small between the inward side and the outward side of the longitudinal outward end 21bleo of the core-wrapping sheet 21b, as described above, the fold position FL is formed at the longitudinal outward end 21leo of the absorbent core 21a (absorbent body 21).

Also, the inward locking member 31A is not overlapped in the longitudinal direction with a region where the leg elastic members 25 exhibit stretchability along the longitudinal direction (the range indicated by dashed lines in FIG. 1). For this reason, in the region where the inward locking member 31A is locked, the contractive force of the leg elastic members 25 is not likely to act on the back sheet 24, and bending and wrinkling of the back sheet 24 is suppressed. Accordingly, the inward locking member 31A can be securely locked to the back sheet 24 in the region where the inward locking member 31A is locked. This therefore makes it possible to further suppress the separation of the belt member 30 from the main body portion 10.

Also, as shown in FIG. 6, the inward locking member 31A is arranged so as to not be overlapped with the widthwise outward end 21weo of the absorbent body 21. Generally, the absorbent body 21 (absorbent core 21a) is formed with a predetermined thickness due to layers of an absorbent material being overlaid on each other in the thickness direction. Accordingly, a height change occurs in the thickness direction at the widthwise outward end 21weo of the absorbent body 21. If the inward locking member 31A is overlapped with the widthwise outward end 21weo of the absorbent body 21, there is a risk that peeling is easily occur at the portion where the height changes in the thickness direction. In contrast, in the present embodiment, the inward locking member 31A is arranged such that the entirety thereof does not cross the outward end 21weo of the absorbent body 21 in the width direction, thus suppressing a problem such as a tendency for peeling to occur at the height change portion.

Furthermore, the inward locking member 31A is arranged outward in the width direction with respect to the outward end 21weo of the absorbent body 21. Specifically, an widthwise inward end 31Awei of the inward locking member 31A is located outward in the width direction with respect to the widthwise outward end 21weo of the absorbent body 21, and the inward locking member 31A is locked at a position not overlapped in the width direction with the absorbent body 21. If the inward locking member 31A is locked at a position overlapped with the absorbent body 21, then when the back portion 7 of the diaper 1 is inserted between a low-weight infant's body and a mat as shown in FIG. 4, the inward locking member 31A becomes sandwiched between the absorbent body 21 and the mat, and thus the inward locking member 31 is forcefully pressed against the mat by the stiff absorbent body 21, and a large amount of frictional force acts on the non-skin-side surface of the inward locking member 31A. In this case, the inward locking member 31A is likely to become peeled away from the back sheet 24 (exterior sheet) as shown in FIG. 4B, and there is a risk that the belt member 30 becomes separated. In contrast, in the present embodiment, the inward locking member 31A and the absorbent body 21 are not overlapped in the width direction, thus suppressing the case where the inward locking member 31A is pressed against the mat by the absorbent body 21 when the diaper 1 is put on. This therefore facilitates suppressing the case where the inward locking member 31A is peeled away from the back sheet 24 (exterior sheet).

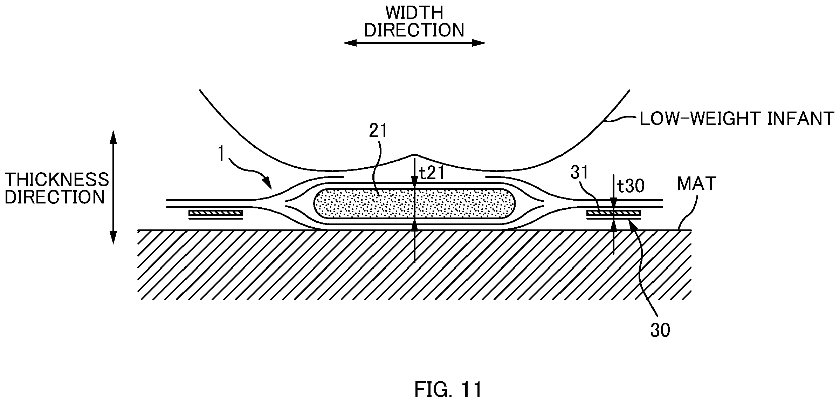

Also, in the diaper 1, a total thickness t30 of the belt member 30 and the locking member 31 (inward locking member 31A) is less than a thickness t21 of the absorbent body 21. FIG. 11 is a schematic cross-sectional view illustrating the thicknesses of portions of the diaper 1. FIG. 11 shows an operation in which the back portion 7 of the diaper 1 is inserted between a low-weight infant's body and a mat when the diaper 1 is being put on the low-weight infant, similarly to the case shown in FIG. 4. As shown in FIG. 11, when the diaper 1 is put on, at least a space (gap in the thickness direction) that corresponds to the thickness t21 of the absorbent body 21 is formed between the low-weight infant's body and the mat. Accordingly, if the total thickness t30 of the belt member 30 and the locking member 31 is less than the thickness t21 of the absorbent body 21 (t30 < t21), the belt member 30 and the locking member 31 fit into the space between the low-weight infant's body and the mat, and therefore the belt member 30 is not likely to come into contact with the mat. Accordingly, friction is not likely to occur between the belt member 30 and the mat, and the case where the locking member 31 becomes peeled away from the exterior sheet as shown in FIG. 4B is likely to be suppressed. This therefore makes it possible to further suppress the separation of the belt member 30.

Also, with respect to the width direction of the diaper 1 in the unfolded and stretched state shown in FIG. 1, a distance W25 between the most outward leg elastic member 25 and the widthwise outward end 24weo of the back sheet 24 (exterior sheet) is less than a width W31A of the locking member 31 (inward locking member 31A). Also, the inward locking member 31A is locked to the back sheet 24 (exterior sheet) such that the widthwise inward end 31Awei of the inward locking member 31A is located inward in the width direction with respect to the most outward leg elastic member 25. If the inward locking member 31A is locked such that the widthwise inward end 31Awei is located outward in the width direction with respect to the most outward leg elastic member 25, the widthwise outward end 31Aweo of the inward locking member 31A becomes located outward in the width direction with respect to the widthwise outward end 24weo of the back sheet 24 (exterior sheet). In other words, a portion of the inward locking member 31A protrudes beyond the outward end 24weo of the back sheet 24 (exterior sheet) in the width direction. In this case, there is a risk that the protruding portion of the inward locking member 31A comes into contact with the low-weight infant (i.e., wearer) and cause the low-weight infant to feel discomfort. In view of this, in the present embodiment, the positional relationship between the inward locking member 31A and the leg elastic members 25 is adjusted such that the inward locking member 31A is not likely to protrude outward beyond the outward end 24weo of the back sheet 24 (exterior sheet) in the width direction. The inward locking member 31A is therefore not likely to come into contact with the low-weight infant's skin.

Also, in the diaper 1 in the unfolded and stretched state shown in FIG. 1, the inward locking member 31A is located outward in the width direction with respect to the LSG elastic member 27. Specifically, the widthwise inward end 31Awei of the inward locking member 31A is located outward in the width direction with respect to the LSG elastic member 27. If the inward locking members 31A are locked to the back sheet 24 so as to be inward in the width direction with respect to the LSG elastic members 27, there is a risk that, when the diaper 1 is put on a low-weight infant as shown in FIG. 3, the longitudinal length of the belt members 30 is insufficient, and it is not possible to lock the outward locking members 31B to the back sheet 24 (exterior sheet) at the stomach region of the low-weight infant. In view of this, in the present embodiment, the lock positions of the inward locking members 31A are prevented from being too far inward in the width direction, thus making it possible for the outward locking members 31B to be reliably locked to the back sheet 24 (exterior sheet) when the diaper 1 is put on.

### Variations of diaper

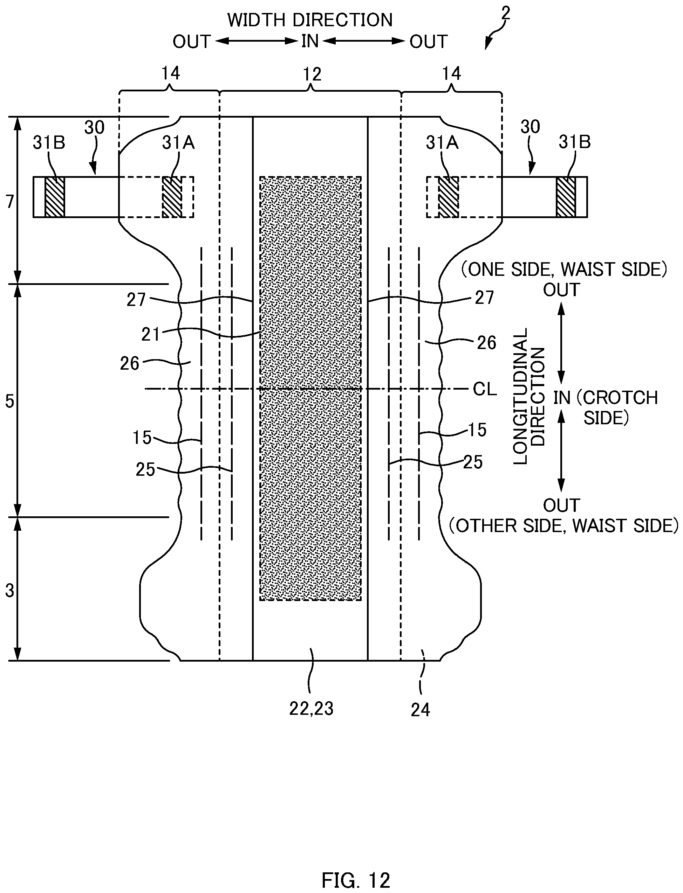

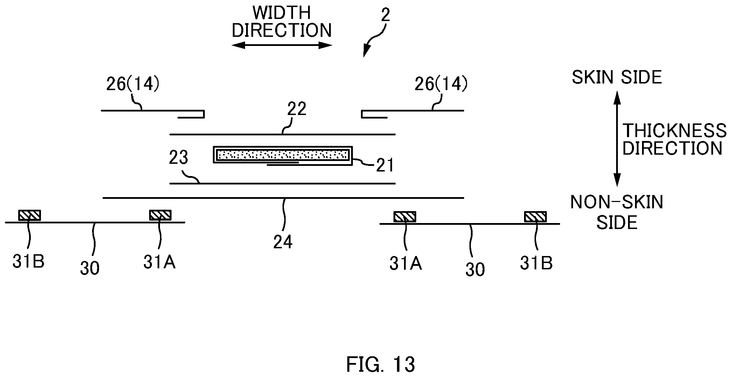

Although the example where the main body portion 10 of the diaper 1 is substantially rectangular as shown in FIG. 1 is described in the above embodiment, the configuration of the diaper 1 may be changed as follows. FIG. 12 is a plan view of a diaper 2 in an unfolded and stretched state, as a variation of the diaper 1. FIG. 13 is an exploded illustrative view of the back portion 7 of the diaper 2.

The main body portion 10 of the diaper 2 has a central band-shaped region 12 and side flaps 14. The central band-shaped region 12 is a band-shaped region that is constituted by the front portion 3, the crotch portion 5, and the back portion 7, and that is located in the central portion in the width direction. The central band-shaped region 12 is the region that corresponds to the main body portion 10 in the diaper 1. The central band-shaped region 12 mainly includes the absorbent body 21, the top sheet 22, the leak-proof sheet 23, and the back sheet 24 (exterior sheet).

The side flaps 14 are portions located at the two widthwise side portions of the central band-shaped region 12. The side flaps 14 extend over the front portion 3, the crotch portion 5, and the back portion 7. The widthwise length (width) of the side flaps 14 in the crotch portion 5 is smaller than the widthwise length (width) of the side flaps 14 in the front portion 3 and the back portion 7. The side flaps 14 are mainly constituted by the side sheets 26 and the back sheet 24 (see FIG. 10).

The pair of side flaps 14 are each provided with side-flap elastic members 15 that stretch and contract in the longitudinal direction. The side-flap elastic members 15 are elastic members such as elastic strings that stretch and contract in the longitudinal direction, and are members that give the leg openings stretchability when the diaper 2 is put on. In other words, the side-flap elastic members 15 are leg elastic members that allow the leg opening portions of the diaper 2 to fit around the wearer's legs. Also, leg gathers are configured by the side-flap elastic members 15 giving stretchability to the side sheets 26 and the back sheet 24 in the crotch portion 5.

In the diaper 2, the belt members 30 are arranged at the two side portions of the side flaps 14 in the width direction in the back portion 7 of the diaper 2 (see FIG. 12). Similarly to the configuration in the diaper 1, the belt members 30 each include locking members 31 (31A, 31B) constituted by hook-and-loop fasteners or the like on respective sides in the longitudinal direction of the belt members 30 (belt longitudinal direction), and the belt members 30 are locked to the non-skin side of the side flaps 14, that is to say the back sheet 24 (exterior sheet), by the inward locking members 31A arranged on the inward side in the width direction of the diaper 2 (one side in the belt longitudinal direction).

The arrangement of the belt member 30 (the lock position of the inward locking member 31A) is similar to that of the diaper 1 described above. Specifically, letting the middle position ML be a position at the middle between the outward end 24leo of the back sheet 24 (exterior sheet) and the outward end 31Aleo of the inward locking member 31A in the longitudinal direction, and letting LY be the distance between the outward end 24leo and the outward end 31Aleo in the longitudinal direction, the belt member 30 is arranged (locked) such that the outward end 21leo of the absorbent body 21 is inward of the middle position ML in the longitudinal direction. Accordingly, when the diaper 2 is put on, a portion of the back sheet 24 folds (24F) and covers the non-skin side of the outward end 31Aleo of the inward locking member 31A, and therefore the inward locking member 31A is not likely to become peeled away from the exterior sheet, and the separation of the belt member 30 from the main body portion 10 is suppressed.

### Other embodiments

Although the embodiment of the present disclosure has been described hereinabove, the above embodiment of the present disclosure is simply to facilitate understanding of the present disclosure and are not in any way to be construed as limiting the present disclosure. The present disclosure may variously be changed or altered without departing from the scope of the claims.

### [Reference Signs List]

1 disposable diaper (diaper),
2 disposable diaper (diaper),
3 front portion, 5 crotch portion, 7 back portion,
10 main body portion,
12 central band-shaped region,
14 side flap,
15 side flap elastic member,
21 absorbent body, 21leo outward end (longitudinal direction), 21weo outward end (width direction),
21a absorbent core, 21b core-wrapping sheet, 21bleo outward end (longitudinal direction),
22 top sheet, 23 leak-proof sheet,
24 back sheet (exterior sheet), 24leo outward end (longitudinal direction), 24F portion,
25 leg elastic member,
26 side sheet,
27 LSG elastic member,
30 belt member,
31 locking member,
31A inward locking member, 31Aleo outward end (longitudinal direction),
31Awei inward end (width direction), 31Aweo outward end (width direction),
31B outward locking member,
100 disposable diaper (comparative example)

## Claims

1. A low-weight infant diaper (1) having a longitudinal direction, a width direction, and a thickness direction that intersect each other,
the low-weight infant diaper comprising:
an absorbent body (21) including at least an absorbent core (21a);
an exterior sheet (24) arranged on a non-skin side with respect to the absorbent body (21); and
a pair of belt members (30) each of which includes a locking member (31A) on a one side in a longitudinal direction of the belt members (30) and that includes a locking member (31B) on another side in the longitudinal direction of the belt members (30),
in a state where each of the belt members (30) is locked, by the one-side locking member (31A), to a non-skin side of the exterior sheet (24) in the thickness direction at a location inward in the longitudinal direction with respect to a longitudinal outward end (24leo) of the exterior sheet (24),
an outward end (21leo) of the absorbent core (21a) is located inward in the longitudinal direction with respect to a middle position (ML),
the middle position (ML) being a position between the longitudinal outward end (24leo) of the exterior sheet (24) and an outward end (31Aleo) of the one-side locking member (31A).

2. The low-weight infant diaper according to claim 1, wherein
the absorbent body (21) includes a core-wrapping sheet (21b) that covers the absorbent core (21a), and
an outward end (21bleo) of the core-wrapping sheet (21b) is located inward in the longitudinal direction with respect to the middle position (ML),
the middle position (ML) being the position between the longitudinal outward end (24leo) of the exterior sheet (24) and the outward end (31Aleo) of the one-side locking member (31A).

3. The low-weight infant diaper according to claim 1 or 2, wherein
the longitudinal outward end (21leo) of the absorbent core (21a) is located in the longitudinal direction between the outward end (31Aleo) of the one-side locking member (31A) and the middle position (ML),
the middle position (ML) being the position between the outward end (24leo) of the exterior sheet (24) and the outward end (31Aleo) of the one-side locking member (31A).

4. The low-weight infant diaper according to claim 1 or 2, wherein
the outward end (21leo) of the absorbent core (21a) is located inward in the longitudinal direction with respect to the outward end (31Aleo) of the one-side locking member (31A).

5. The low-weight infant diaper according to any one of claims 1 to 4, wherein
the low-weight infant diaper (1) further comprises a leg elastic member (25) capable of stretching and contracting in the longitudinal direction, and
the one-side locking member (31A) is not overlapped in the longitudinal direction with a region in which the leg elastic member (25) exhibits stretchability.

6. The low-weight infant diaper according to any one of claims 1 to 5, wherein
the one-side locking member (31A) is not overlapped in the width direction with an outward end (21weo) of the absorbent body (21).

7. The low-weight infant diaper according to claim 6, wherein
an inward end (31Awei) of the one-side locking member (31A) is located outward in the width direction with respect to the outward end (21weo) of the absorbent body (21).

8. The low-weight infant diaper according to any one of claims 1 to 7, wherein
a total of a thickness of each of the belt members (30) and a thickness of the one-side locking member (31A) is less than a thickness of the absorbent body (21).

9. The low-weight infant diaper according to any one of claims 1 to 8, wherein
the low-weight infant diaper (1) further comprises a leg elastic member (25) capable of stretching and contracting in the longitudinal direction,
a widthwise distance (W25) between an outermost leg elastic member (25) and a widthwise outward end (24weo) of the exterior sheet (24) is less than a width (W31A) of the one-side locking member (31A), and
a widthwise inward end portion (31Awei) of the one-side locking member (31A) is located inward in the width direction with respect to the outermost leg elastic member (25).

10. The low-weight infant diaper according to any one of claims 1 to 9, wherein
the low-weight infant diaper (1) further comprises a pair of leak-proof walls on respective widthwise sides of the absorbent body (21),
each of the leak-proof walls includes an LSG elastic member (27) capable of stretching and contracting in the longitudinal direction, and
in a state where the low-weight infant diaper has been unfolded and stretched,
the one-side locking member (31A) is located outward in the width direction with respect to the LSG elastic members (27).

## Patentansprüche

1. Leichtgewichtige Säuglingswindel (1), die eine Längsrichtung, eine Breitenrichtung und eine Dickenrichtung aufweist, die einander schneiden,
wobei die leichtgewichtige Säuglingswindel Folgendes umfasst:
einen Saugkörper (21), der mindestens einen Saugkern (21a) einschließt;
eine äußere Lage (24), die auf einer Nicht-Hautseite in Bezug auf den Saugkörper (21) angeordnet ist; und
ein Paar von Gürtelelementen (30), von denen jedes ein Verschlusselement (31A) auf einer Seite in einer Längsrichtung der Gürtelelemente (30) einschließt und das ein Verschlusselement (31B) auf einer anderen Seite in der Längsrichtung der Gürtelelemente (30) einschließt,
wobei in einem Zustand, wo jedes der Gürtelelemente (30) durch das einseitige Verschlusselement (31A) mit einer Nicht-Hautseite der äußeren Lage (24) in der Dickenrichtung an einer Stelle innen in der Längsrichtung in Bezug auf ein längsgerichtetes äußeres Ende (24leo) der äußeren Lage (24) verschlossen ist,
sich ein äußeres Ende (21leo) des Saugkerns (21a) innen in der Längsrichtung in Bezug auf eine mittlere Position (ML) befindet,
wobei die mittlere Position (ML) eine Position zwischen dem längsgerichteten äußeren Ende (24leo) der äußeren Lage (24) und einem äußeren Ende (31Aleo) des einseitigen Verschlusselements (31A) ist.

2. Leichtgewichtige Säuglingswindel nach Anspruch 1, wobei
der Saugkörper (21) eine den Kern umhüllende Lage (21b) einschließt, die den Saugkern (21a) bedeckt, und
sich ein äußeres Ende (21bleo) der den Kern umhüllenden Lage (21b) innen in der Längsrichtung in Bezug auf die mittlere Position (ML) befindet,
wobei die mittlere Position (ML) die Position zwischen dem längsgerichteten äußeren Ende (24leo) der äußeren Lage (24) und dem äußeren Ende (31Aleo) des einseitigen Verschlusselements (31A) ist.

3. Leichtgewichtige Säuglingswindel nach Anspruch 1 oder 2, wobei
sich das längsgerichtete äußere Ende (21leo) des Saugkerns (21a) in der Längsrichtung zwischen dem äußeren Ende (31Aleo) des einseitigen Verschlusselements (31A) und der mittleren Position (ML) befindet,
wobei die mittlere Position (ML) die Position zwischen dem äußeren Ende (24leo) der äußeren Lage (24) und dem äußeren Ende (31Aleo) des einseitigen Verschlusselements (31A) ist.

4. Leichtgewichtige Säuglingswindel nach Anspruch 1 oder 2, wobei
sich das äußere Ende (21leo) des Saugkerns (21a) innen in der Längsrichtung in Bezug auf das äußere Ende (31Aleo) des einseitigen Verschlusselements (31A) befindet.

5. Leichtgewichtige Säuglingswindel nach einem der Ansprüche 1 bis 4, wobei
die leichtgewichtige Säuglingswindel (1) weiter ein elastisches Beinelement (25) umfasst, das sich in der Längsrichtung dehnen und zusammenziehen kann, und
das einseitige Verschlusselement (31A) in der Längsrichtung nicht mit einem Bereich überlappt, in dem das elastische Beinelement (25) Dehnbarkeit besitzt.

6. Leichtgewichtige Säuglingswindel nach einem der Ansprüche 1 bis 5, wobei
das einseitige Verschlusselement (31A) in der Breitenrichtung nicht mit einem äußeren Ende (21weo) des Saugkörpers (21) überlappt.

7. Leichtgewichtige Säuglingswindel nach Anspruch 6, wobei
sich ein inneres Ende (31Awei) des einseitigen Verschlusselements (31A) außen in der Breitenrichtung in Bezug auf das äußere Ende (21weo) des Saugkörpers (21) befindet.

8. Leichtgewichtige Säuglingswindel nach einem der Ansprüche 1 bis 7, wobei
insgesamt eine Dicke von jedem der Gürtelelemente (30) und eine Dicke des einseitigen Verschlusselements (31A) kleiner ist als eine Dicke des Saugkörpers (21).

9. Leichtgewichtige Säuglingswindel nach einem der Ansprüche 1 bis 8, wobei
die leichtgewichtige Säuglingswindel (1) weiter ein elastisches Beinelement (25) umfasst, das sich in der Längsrichtung dehnen und zusammenziehen kann,
ein Abstand in der Breite (W25) zwischen einem äußersten elastischen Beinelement (25) und einem äußeren Ende in der Breite (24weo) der äußeren Lage (24) kleiner ist als eine Breite (W31A) des einseitigen Verschlusselements (31A) und
sich ein innerer Endteil in der Breite (31Awei) des einseitigen Verschlusselements (31A) innen in der Breitenrichtung in Bezug auf das äußerste elastische Beinelement (25) befindet.

10. Leichtgewichtige Säuglingswindel nach einem der Ansprüche 1 bis 9, wobei
die leichtgewichtige Säuglingswindel (1) weiter ein Paar von auslaufsicheren Wänden auf jeweiligen Seiten in der Breite des Saugkörpers (21) umfasst,
jede der auslaufsicheren Wände ein elastisches LSG-Element (27) einschließt, das sich in der Längsrichtung dehnen und zusammenziehen kann, und
in einem Zustand, wo die leichtgewichtige Säuglingswindel entfaltet und gedehnt wurde,
sich das einseitige Verschlusselement (31 A) außen in der Breitenrichtung in Bezug auf die elastischen LSG-Elemente (27) befindet.

## Revendications

1. Couche pour nourrisson de faible poids (1) ayant une direction allant dans le sens longitudinal, une direction allant dans le sens de la largeur, et une direction allant dans le sens de l'épaisseur qui se croisent les unes les autres,
la couche pour nourrisson de faible poids comportant :
un corps absorbant (21) comprenant au moins une partie centrale absorbante (21a) ;
une feuille extérieure (24) agencée sur un côté non orienté vers la peau par rapport au corps absorbant (21) ; et
une paire d'éléments formant ceinture (30), chacun d'entre eux comprenant un élément de blocage (31A) se trouvant sur un côté dans une direction allant dans le sens longitudinal des éléments formant ceinture (30) et comprenant un élément de blocage (31B) sur un autre côté dans la direction allant dans le sens longitudinal des éléments formant ceinture (30),
dans un état dans lequel chacun des éléments formant ceinture (30) est bloqué, par l'élément de blocage se trouvant d'un côté (31A), sur un côté non orienté vers la peau de la feuille extérieure (24) dans la direction allant dans le sens de l'épaisseur au niveau d'un emplacement vers l'intérieur dans la direction allant dans le sens longitudinal par rapport à une extrémité allant vers l'extérieur dans le sens longitudinal (24leo) de la feuille extérieure (24),
une extrémité allant vers l'extérieur (21leo) de la partie centrale absorbante (21a) est située vers l'intérieur dans la direction allant dans le sens longitudinal par rapport à une position intermédiaire (ML),
la position intermédiaire (ML) étant une position entre l'extrémité allant vers l'extérieur dans le sens longitudinal (24leo) de la feuille extérieure (24) et une extrémité allant vers l'extérieur (31Aleo) de l'élément de blocage se trouvant d'un côté (31A).

2. Couche pour nourrisson de faible poids selon la revendication 1, dans laquelle
le corps absorbant (21) comprend une feuille d'enveloppement de partie centrale (21b) qui recouvre la partie centrale absorbante (21a), et
une extrémité allant vers l'extérieur (21bleo) de la feuille d'enveloppement de partie centrale (21b) est située vers l'intérieur dans la direction allant dans le sens longitudinal par rapport à la position intermédiaire (ML),
la position intermédiaire (ML) étant la position entre l'extrémité allant vers l'extérieur dans le sens longitudinal (24leo) de la feuille extérieure (24) et l'extrémité allant vers l'extérieur (31Aleo) de l'élément de blocage se trouvant d'un côté (31A).

3. Couche pour nourrisson de faible poids selon la revendication 1 ou la revendication 2, dans laquelle
l'extrémité allant vers l'extérieur dans le sens longitudinal (21leo) de la partie centrale absorbante (21a) est située dans la direction allant dans le sens longitudinal entre l'extrémité allant vers l'extérieur (31Aleo) de l'élément de blocage se trouvant d'un côté (31A) et la position intermédiaire (ML),
la position intermédiaire (ML) étant la position entre l'extrémité allant vers l'extérieur (24leo) de la feuille extérieure (24) et l'extrémité allant vers l'extérieur (31Aleo) de l'élément de blocage se trouvant d'un côté (31A).

4. Couche pour nourrisson de faible poids selon la revendication 1 ou la revendication 2, dans laquelle
l'extrémité allant vers l'extérieur (21leo) de la partie centrale absorbante (21a) est située vers l'intérieur dans la direction allant dans le sens longitudinal par rapport à l'extrémité allant vers l'extérieur (31Aleo) de l'élément de blocage se trouvant d'un côté (31A).

5. Couche pour nourrisson de faible poids selon l'une quelconque des revendications 1 à 4, dans laquelle
la couche pour nourrisson de faible poids (1) comporte par ailleurs un élément élastique pour jambe (25) en mesure de s'étirer et de se contracter dans la direction allant dans le sens longitudinal, et
l'élément de blocage se trouvant d'un côté (31A) n'est pas chevauché dans la direction allant dans le sens longitudinal par une région dans laquelle l'élément élastique pour jambe (25) présente une aptitude à l'extensibilité.

6. Couche pour nourrisson de faible poids selon l'une quelconque des revendications 1 à 5, dans laquelle
l'élément de blocage se trouvant d'un côté (31A) n'est pas chevauché dans la direction allant dans le sens de la largeur par une extrémité allant vers l'extérieur (21weo) du corps absorbant (21).

7. Couche pour nourrisson de faible poids selon la revendication 6, dans laquelle
une extrémité allant vers l'intérieur (31Awei) de l'élément de blocage se trouvant d'un côté (31A) est située vers l'extérieur dans la direction allant dans le sens de la largeur par rapport à l'extrémité allant vers l'extérieur (21weo) du corps absorbant (21).

8. Couche pour nourrisson de faible poids selon l'une quelconque des revendications 1 à 7, dans laquelle
un total d'une épaisseur de chacun des éléments formant ceinture (30) et d'une épaisseur de l'élément de blocage se trouvant d'un côté (31A) est inférieur à une épaisseur du corps absorbant (21).

9. Couche pour nourrisson de faible poids selon l'une quelconque des revendications 1 à 8, dans laquelle
la couche pour nourrisson de faible poids (1) comporte par ailleurs un élément élastique pour jambe (25) en mesure de s'étirer et de se contracter dans la direction allant dans le sens longitudinal,
une distance dans le sens de la largeur (W25) entre un élément élastique pour jambe se trouvant le plus à l'extérieur (25) et une extrémité allant vers l'extérieur dans le sens de la largeur (24weo) de la feuille extérieure (24) est inférieure à une largeur (W31A) de l'élément de blocage se trouvant d'un côté (31A), et
une partie d'extrémité allant vers l'intérieur dans le sens de la largeur (31Awei) de l'élément de blocage se trouvant d'un côté (31A) est située vers l'intérieur dans la direction allant dans le sens de la largeur par rapport à l'élément élastique pour jambe se trouvant le plus à l'extérieur (25).

10. Couche pour nourrisson de faible poids selon l'une quelconque des revendications 1 à 9, dans laquelle
la couche pour nourrisson de faible poids (1) comporte par ailleurs une paire de parois antifuite sur des côtés respectifs dans le sens de la largeur du corps absorbant (21),
chacune des parois antifuite comprend un élément élastique LSG (27) en mesure de s'étirer et de se contracter dans la direction allant dans le sens longitudinal, et
dans un état dans lequel la couche pour nourrisson de faible poids a été dépliée et étirée,
l'élément de blocage se trouvant d'un côté (31A) est situé vers l'extérieur dans la direction allant dans le sens de la largeur par rapport à l'élément élastique LSG (27).
